# EUROPEAN PATENT SPECIFICATION

(11) **EP 2 859 878 B1**
(45) Date of publication and mention of the grant of the patent: **13.09.2017**
(21) Application number: 14175935.7
(22) Date of filing: 07.07.2014
(51) Int. Cl.: A61K 8/29, A61K 8/73, A61K 8/97, A61K 8/02, A61K 8/19, A61K 8/55, A61Q 19/00

(54) **Method of preparing a cream for cutaneous application**
Verfahren zur Herstellung einer Creme zur Anwendung auf der Haut
Procédé de préparation d'une crème pour application cutanée

(30) Priority: 08.07.2013 ES 201331034
(43) Date of publication of application: 15.04.2015
(73) Proprietor: Procoluide Industrial, S.A.U., 28010 Madrid (ES)
(72) Inventor: Redondo Hernandez, Vicente, 28010 Madrid (ES); Barrios Rodriguez, Javier, 28010 Madrid (ES)
(74) Representative: Hart-Davis, Jason

(56) References cited:
- EP-A1- 0 446 290
- US-A- 4 622 074
- US-A- 5 798 109
- US-A1- 2007 020 209

## Description

### Field of Art

The present invention relates to cosmetics for topical application on the skin, particularly on the face, providing luminosity, moisture, color and protection against the harmful effects of solar radiation, proposing to that end a method of preparing a cream for cutaneous application that allows obtaining a wide variety of colors being suited to every user's needs and with a less noticeable color in the cream itself, more specifically a substantially white color.

### State of the Art

Women use a wide range of products in their daily makeup routine. First they apply a moisturizing product, for example a moisturizing cream, on their face providing moisture to the skin. They apply a foundation over the moisturizing product and then apply conventional makeup over said foundation. Additionally, they usually apply sunscreens to protect the skin against the damaging effects of solar radiation, perfumes or other cosmetic active ingredients.

To prevent having to apply all these products consecutively in the daily makeup routine, with the time that it entails, creams known on the market as BB Cream, which are colored creams providing the skin with moisture, color and protection against solar radiation in a single product, are known.

Such creams have a very noticeable color in the cream itself, generally having grayish or brownish tones. This causes certain rejection of the cream by users for certain aesthetic reasons, creams having a color that is as white as possible being more appealing on the market.

These creams have a very noticeable color in the cream itself because the pigments, generally iron oxides, intended for providing the desired color tone to the user during cream application end up contributing their color to the cream during the process of manufacturing said cream. In the process of manufacturing these creams, the cream is first prepared in an oil-water emulsion, and pigments which will be encapsulated with polymers are finally added thereafter. For the pigments to be homogenously distributed in the cream, and since the oil-water emulsion of the cream is a rather viscous composition, the emulsion with pigments therein must be subjected to high-energy stirring, which can cause the pigment encapsulation to break and end up contributing color to the cream.

On the other hand, manufacturers of such creams purchase the pigments directly from different suppliers. These pigments are pre-encapsulated and standardized to a very limited range of colors; therefore, directly adding these encapsulated pigments at the end of the process of preparing the cream does not allow obtaining creams that adapt to every user's needs.

Documents US 2007/0020209 A1 and WO2011006657 disclose makeup compositions in the form of a water-oil or oil-water cosmetic emulsion wherein the pigments contributing color to the cream are added in pre-encapsulated form at the end of the method of preparing the cream, whereby obtaining a composition with a noticeable color in the cream itself before application, as well as a very limited range of colors.

US Patent 4,622,074 shows pigments or extender pigments the surface of which are uniformly coated with hydrogenated lecithin or with the reaction product of hydrogenated lecithin and a metal salt in a composition for make-up cosmetics.

It is therefore necessary to have a cream which contributes moisture, luminosity, color and solar protection, but with a less noticeable color or a white color in the cream, and which allows obtaining a color suited to every user's needs.

### Object of the Invention

A cream for cutaneous application with a white color in the cream itself providing luminosity, moisture, color and protection against the harmful effects of solar radiation, and with a range of colors is suited to every user's needs.

The invention proposes a method of preparing a cream for cutaneous application which is made up of a cream base consisting of a water-oil emulsion to which a series of water-based cosmetic active ingredients is added to obtain a cream for cutaneous application consisting of a triple water-oil-water emulsion.

The method of preparing the cream base consisting of a water-oil emulsion comprises the steps of:
- Selecting a variety of iron oxide pigments.
- Dispersing the selected iron oxide pigments, for example by means of a mill, until obtaining a homogenous dispersion according to the desired color for the cream.
- Mixing the iron oxide pigments with cellulose in the aqueous phase until obtaining a homogenous mixture.
- Mixing vegetable oil and hydrogenated lecithin-coated titanium oxide particles in the oily phase.
- Emulsifying the aqueous phase with the oily phase, leaving to stand at room temperature.

Iron oxide pigments range between 0.01-5%, cellulose ranges between 0.1-2%, hydrogenated lecithin-coated titanium oxide particles range between 2-10%, vegetable oil ranges between the 10-40%, all percentages being presented by weight in relation to the total weight of the cream base.

In the aqueous phase where the iron oxide pigments are mixed with cellulose, magnesium sulfate, glycerin and a preservative are additionally mixed in. Magnesium sulfate ranges between 1-2%, glycerin ranges between 1-4% and the preservative ranges between 0.1-2%, all percentages being presented by weight in relation to the total weight of the cream base.

An emulsifier is added to the aqueous phase thus formed under stirring and is heated at a temperature comprised between 55-60°C. The aqueous phase is subsequently emulsified with the oily phase under stirring at a temperature comprised between 55-60°C.

Partially delaminated mica is added to the emulsion of the aqueous phase with the oily phase in a proportion ranging between 1-8% by weight in relation to the total weight of the cream base, which intensifies the white color of the cream base. The emulsion thus formed is left to stand at room temperature for at least 24 hours.

Unlike conventional processes of manufacturing creams for cutaneous application where the pre-encapsulated iron oxide pigments are incorporated when the process of manufacturing the cream ends, with the subsequent risk of the pigment encapsulation breaking, the invention proposes encapsulating iron oxide pigments directly in the process of manufacturing the cream. Iron oxide pigments are thus incorporated in the aqueous phase, being homogenously dispersed in said aqueous phase and correctly encapsulated by the cellulose. Since the iron oxide pigments are already homogenously distributed in the aqueous phase when the aqueous phase is emulsified with the oily phase, the need to subject the emulsion to high-energy stirring in order to homogenously distribute the pigments like in conventional methods is avoided, thus preventing the iron oxide pigment encapsulation from being able to break, contributing color to the cream.

The iron oxide pigments are selected from the group comprising red iron oxide, black iron oxide, yellow iron oxide, brown iron oxide, russet iron oxide and orange iron oxide, such that by combining the selected iron oxides and the amount in which they are added, a color on the skin suited to every user's specific needs is obtained, unlike conventional solutions in which oxide pigments were added to the cream in pre-encapsulated form according to standard colors.

A cream with a white color in the cream itself, which is unlike creams existing on the market which directly contribute color to the cream, is thus obtained. The cream of the invention is white because the iron oxide pigments responsible for contributing color to the user's skin are encapsulated in the aqueous phase during the actual process of preparing the cream, without contributing color to same. Therefore, when the cream is applied on the user's skin applying slight pressure using her fingers, the encapsulated iron oxide pigments break and spread over the skin, resulting in the required and homogenous tone. On the other hand, by selecting and encapsulating the pigments in the actual process of preparing the cream, the required color tone can be obtained.

### Detailed Description of the Invention

The cream obtained with the method of preparing of the invention is made up of a cream base to which a series of widely accepted cosmetic active ingredients contributing different functions, such as solar protection, cellular protection, anti-spot and depigmentation effects, skin correction, antioxidant power, moisturization and revitalizing effect, are added.

The cream base consists of an aqueous phase and an oily phase which are mixed forming a water-oil emulsion according to claim 1. A variety of iron oxide pigments, selected from the group comprising red iron oxide, black iron oxide, yellow iron oxide, brown iron oxide, russet iron oxide, orange iron oxide and mixtures thereof, are added to the aqueous phase. Depending on the type of pigments that are selected and on the amount in which they are added to the aqueous phase, an end product with different color tones is obtained.

Unlike conventional solutions, in the present invention iron oxide pigments are encapsulated in cellulose or cellulose derivatives in the aqueous phase during the actual process of preparing the cream base. Cellulose has a high iron oxide adhesion power and envelops said iron oxides, preventing them from contributing color to the cream, and the cellulose envelope also prevents accidental iron oxide dispersion during the process of preparing the cream. The iron oxides are thus enveloped by the cellulose until the cream is applied on the skin by applying slight pressure using the fingers, at which time the cellulose envelope breaks and the iron oxides are released, contributing the desired color.

The aqueous phase of the cream base additionally has magnesium sulfate, glycerin, a preservative and water, such that the iron oxide pigments are in dispersion in the aqueous phase and form a homogenous suspension with it.

The oily phase of the cream base consists of a vegetable oil, preferably macadamia oil, and hydrogenated lecithin-coated titanium oxide particles. Titanium oxide (TiO₂) has great whitening power, resulting in the substantially white aesthetic appearance of the cream. The hydrogenated lecithin surrounding the titanium oxide particles has a hydrophobic residue, such that the hydrogenated lecithin has affinity for the vegetable oil in which it is incorporated, the apolar chains of lecithin facing outwards due to their affinity for oil and the polar fragments facing inwards due to their affinity for titanium oxide. The hydrogenated lecithin-coated titanium oxide particles are thus in dispersion in the oily phase forming a homogenous suspension with it.

The oily phase and the aqueous phase of the cream base are mixed under stirring to form a water-oil emulsion, such that since the iron oxide pigments are already in dispersion in the aqueous phase and form a homogenous suspension with it, accidental breaks of the cellulose encapsulation surrounding the pigments do not occur, said pigments being prevented from being able to contribute color to the cream base.

Partially delaminated mica is added to this water-oil emulsion. Since the mica is partially delaminated, it has a series of cavities such that the emulsion of the oily phase with the aqueous phase is gradually placed in the cavities of the mica, so the iron oxide pigments surrounded by cellulose are additionally protected in said cavities. Furthermore, mica intensifies the whitening of the cream base and improves cream application on the skin since it is a very smooth material. The iron oxide pigments are therefore enveloped in the cellulose and dispersed in the cream, not contributing color to the cream until the time of application thereof on the skin. Furthermore, these iron oxide pigments are visually concealed in the cream base, such that the cream has a substantially white color. This is because when the aqueous phase is emulsified with the white oily phase, the entire emulsion has a white color, and the subsequent addition of the mica helps to contribute more white color to the cream base.

Finally, a series of cosmetic active ingredients are added on the cream base in an aqueous phase, such that the finally obtained product is a cream for cutaneous application in the form of a triple water-oil-water emulsion. That being the case, there is added to the cream base an active ingredient providing solar protection, an active ingredient with an anti-glycation effect protecting cell DNA from impairments caused by sunlight, a natural active ingredient reducing melanocyte proliferation and acting as a depigmenting agent preventing the occurrence of new spots, an active ingredient with unifying action correcting imperfections and leaving the skin smooth and glowing, vitamins, A, C and E contributing antioxidant power, an algae extract rich in omega-3 and omega-6 having moisturizing properties, and a pearl extract with revitalizing and remineralizing properties.

The following example shows a possible non-limiting embodiment of the cream base. The amounts are indicated in % by weight in relation to the total weight of the cream base.

| Ingredients | % by weight |
|---|---|
| Red iron oxide | 0.01 - 1.0 |
| Black iron oxide | 0.01 - 1.0 |
| Yellow iron oxide | 0.01 - 3.0 |
| Hydrogenated lecithin-coated titanium oxide | 2.0 - 10.0 |
| Macadamia oil | 10.0 - 40.0 |
| Cellulose-derived gel | 0.1 - 2.0 |
| Magnesium sulfate | 1.0 - 2.0 |
| Glycerin | 1.0 - 4.0 |
| Water | qs 100 |
| Preservative | 0.1 - 2.0 |
| Emulsifier | 1.0 - 8.0 |
| Mica | 1.0 - 8.0 |

The preceding ingredients are combined to form an oil-water emulsion. First, preliminary operations are carried out in which the selected iron oxides are dispersed in a mill until obtaining a homogenous dispersion according to the desired color for the cream. In the present example, the selected iron oxides are red, black and yellow iron oxides, in respective proportions of 0.01 - 1.0%, 0.01 - 1.0%, and 0.01-3.0% by weight in relation to the total weight of the cream base.

The magnesium sulfate, glycerin, water, preservative and cellulose-derived gel are mixed in a reactor at room temperature and under stirring. On the other hand, the vegetable oil and hydrogenated lecithin-coated titanium oxide particles are mixed in a main tank under stirring, resulting in the oily phase.

The iron oxides are then mixed under stirring with the mixture of magnesium sulfate, glycerin, water, preservative and cellulose-derived gel, resulting in the aqueous phase. An emulsifier is added to the aqueous phase under stirring and is heated at a temperature comprised between 55-60°C. On the other hand, the oily phase consisting of vegetable oil and hydrogenated lecithin-coated titanium oxide particles is heated at a temperature comprised between 55-60°C and added to the main tank under stirring, the aqueous phase on the oily phase, homogenizing the water-oil emulsion thus formed with a marine propeller type stirrer or another suitable stirrer. Partially delaminated mica is added to this finally formed water-oil emulsion. Once the cream base preparation ends, it is cooled down at room temperature, leaving it to stand for at least 24 hours.

The cream base has a proportion in the order of 70-80% by weight in relation to the total weight of the cream, such that the finally prepared cream has a substantially white color unlike other similar creams existing on the market.

Once the cream base consisting of the water-oil emulsion has settled, a second aqueous phase is added under stirring to form a triple water-oil-water emulsion. A series of cosmetic active ingredients widely used in the preparation of creams for cutaneous application are added in this second aqueous phase. Xanthan gum, which is used as a stabilizing agent for the water-oil-water emulsion, and non-ionic emulsifiers, such as decyl glucoside, an active ingredient for solar protection which, besides acting as a filter for solar protection also acts as a stabilizing agent for the second aqueous phase of the emulsion, are added in this second aqueous phase under stirring. Given that the external phase in the final cream, which is a water-oil-water emulsion, is aqueous, a texture of cream that is very pleasant to the touch when applying said cream is achieved.

## Claims

1. A method of preparing a cream for releasing iron oxides thereby developing a color upon cutaneous application, comprising a cream base consisting of an aqueous phase and an oily phase, **characterized in that** preparing the cream base comprises the steps of:
- selecting a variety of iron oxide pigments,
- dispersing the selected iron oxide pigments,
- mixing the iron oxide pigments with cellulose in the aqueous phase until obtaining a homogenous mixture,
- mixing vegetable oil and hydrogenated lecithin-coated titanium oxide particles in the oily phase,
- emulsifying the aqueous phase with the oily phase, leaving to stand at room temperature.

2. The method of preparing a cream according to claim 1, **characterized in that** the iron oxide pigments are selected from the group comprising red iron oxide, black iron oxide, yellow iron oxide, brown iron oxide, russet iron oxide and orange iron oxide.

3. The method of preparing a cream according to claim 1, **characterized in that** the iron oxide pigments range between 0.01-5%, cellulose ranges between 0.1-2%, hydrogenated lecithin-coated titanium oxide particles range between 2-10%, vegetable oil ranges between 10-40%, all percentages being presented by weight in relation to the total weight of the cream base.

4. The method of preparing a cream according to claim 1, **characterized in that** an emulsifier is added to the aqueous phase under stirring and is heated at a temperature comprised between 55-60°C.

5. The method of preparing a cream according to claim 1, **characterized in that** the aqueous phase is emulsified with the oily phase under stirring at a temperature comprised between 55-60°C.

6. The method of preparing a cream according to claim 1, **characterized in that** magnesium sulfate, glycerin and a preservative are additionally mixed in the aqueous phase.

7. The method of preparing a cream according to the preceding claim, **characterized in that** magnesium sulfate ranges between 1-2%, glycerin ranges between 1-4% and the preservative ranges between 0.1-2%, all percentages being presented by weight in relation to the total weight of the cream base.

8. The method of preparing a cream according to claim 1, **characterized in that** partially delaminated mica is added to the emulsion of the aqueous phase with the oily phase.

9. The method of preparing a cream according to the preceding claim, **characterized in that** partially delaminated mica ranges between 1-8% by weight in relation to the total weight of the cream base.

10. The method of preparing a cream according to claim 1, **characterized in that** the vegetable oil added in the oily phase is macadamia oil.

11. The method of preparing a cream according to any of the preceding claims, comprising
- settling the emulsion of the aqueous phase with the oily phase and thereafter adding under stirring a cosmetic active ingredient in a second aqueous phase to form a triple water-oil-water emulsion.

## Patentansprüche

1. Verfahren zum Herstellen einer Creme zum Freisetzen von Eisenoxiden, wodurch bei kutaner Anwendung eine Farbe entwickelt wird, umfassend einen Cremegrundstoff, der aus einer wässrigen Phase und einer öligen Phase besteht, **dadurch gekennzeichnet, dass** das Herstellen des Cremegrundstoffes die Schritte umfasst:
- Auswählen einer Vielzahl von Eisenoxidpigmenten,
- Dispergieren der ausgewählten Eisenoxidpigmente,
- Mischen der Eisenoxidpigmente mit Cellulose in der wässrigen Phase, bis eine homogene Mischung erhalten wird,
- Mischen eines Pflanzenöls und hydrogenierter lecithinbeschichteter Titanoxidpartikel in der öligen Phase,
- Emulgieren der wässrigen Phase und der öligen Phase, Stehenlassen bei Raumtemperatur.

2. Verfahren zum Herstellen einer Creme nach Anspruch 1, **dadurch gekennzeichnet, dass** die Eisenoxidpigmente aus der aus rotem Eisenoxid, schwarzem Eisenoxid, gelbem Eisenoxid, braunem Eisenoxid, rostbraunem Eisenoxid und orangenem Eisenoxid bestehenden Gruppe ausgewählt sind.

3. Verfahren zum Herstellen einer Creme nach Anspruch 1, **dadurch gekennzeichnet, dass** die Eisenoxidpigmente in einem Bereich von 0,01 - 5 % vorliegen, die Cellulose in einem Bereich von 0,1 - 2 %vorliegt, die hydrogenierten lecithinbeschichteten Titanoxidpartikel in einem Bereich von 2 - 10 % vorliegen, das Pflanzenöl in einem Bereich von 10 - 40 % vorliegt, wobei alle Prozentangaben als Gewichtsprozent in Relation zum Gesamtgewicht des Cremegrundstoffes angegeben sind.

4. Verfahren zum Herstellen einer Creme nach Anspruch 1, **dadurch gekennzeichnet, dass** ein Emulgator zur wässrigen Phasen unter Rühren hinzugefügt und auf eine Temperatur zwischen 55 - 60 °C erhitzt wird.

5. Verfahren zum Herstellen einer Creme nach Anspruch 1, **dadurch gekennzeichnet, dass** die wässrige Phase und die ölige Phase unter Rühren bei einer Temperatur zwischen 55 - 60 °C emulgiert werden.

6. Verfahren zum Herstellen einer Creme nach Anspruch 1, **dadurch gekennzeichnet, dass** Magnesiumsulfat, Glycerin und ein Konservierungsmittel zusätzlich in die wässrige Phase gemischt werden.

7. Verfahren zum Herstellen einer Creme nach dem vorhergehenden Anspruch, **dadurch gekennzeichnet, dass** Magnesiumsulfat in einem Bereich von 1 - 2 % vorliegt, Glycerin in einem Bereich von 1 - 4 %vorliegt und das Konservierungsmittel in einem Bereich von 0,1 - 2 % vorliegt, wobei alle Prozentangaben als Gewichtsprozent in Relation zum Gesamtgewicht des Cremegrundstoffes angegeben sind.

8. Verfahren zum Herstellen einer Creme nach Anspruch 1, **dadurch gekennzeichnet, dass** zur Emulsion aus der wässrigen Phase und der öligen Phase teilweise delaminierter Glimmer hinzugefügt wird.

9. Verfahren zum Herstellen einer Creme nach dem vorhergehenden Anspruch, **dadurch gekennzeichnet, dass** der teilweise delaminierte Glimmer in einem Bereich von 1 - 8 Gewichts-% in Relation zum Gesamtgewicht des Cremegrundstoffes vorliegt.

10. Verfahren zum Herstellen einer Creme nach Anspruch 1, **dadurch gekennzeichnet, dass** das in der öligen Phase hinzugefügte Pflanzenöl Macadamiaöl ist.

11. Verfahren zum Herstellen einer Creme nach einem der vorhergehenden Ansprüche, umfassend
- Absetzen der Emulsion aus der wässrigen Phase und der öligen Phase und danach unter Rühren Hinzufügen eines kosmetisch aktiven Inhaltsstoffes in einer zweiten wässrigen Phase, um eine Triple-Wasser-Öl-Wasser-Emulsion zu bilden.

## Revendications

1. Procédé de préparation d'une crème pour libérer des oxydes de fer développant par-là une couleur par application cutanée, comprenant une base de crème consistant en une phase aqueuse et une phase huileuse, **caractérisé en ce que** la préparation de la base de crème comprend les étapes de :
- choix d'une variété de pigments d'oxyde de fer,
- dispersion des pigments d'oxyde de fer choisis,
- mélange des pigments d'oxyde de fer avec de la cellulose dans la phase aqueuse jusqu'à l'obtention d'un mélange homogène,
- mélange d'huile végétale et de particules d'oxyde de titane revêtues de lécithine hydrogénée dans la phase huileuse,
- émulsification de la phase aqueuse avec la phase huileuse, mise au repos à température ambiante.

2. Procédé de préparation d'une crème selon la revendication 1, **caractérisé en ce que** les pigments d'oxyde de fer sont choisis dans le groupe comprenant de l'oxyde de fer rouge, de l'oxyde de fer noir, de l'oxyde de fer jaune, de l'oxyde de fer brun, de l'oxyde de fer rouille et de l'oxyde de fer orange.

3. Procédé de préparation d'une crème selon la revendication 1, **caractérisé en ce que** les pigments d'oxyde de fer sont entre 0,01-5 %, la cellulose entre 0,1-2 %, les particules d'oxyde de titane revêtues de lécithine hydrogénée entre 2-10 %, l'huile végétale entre 10-40 %, tous les pourcentages étant présentés en masse par rapport à la masse totale de la base de crème.

4. Procédé de préparation d'une crème selon la revendication 1, **caractérisé en ce qu'**un émulsionnant est ajouté à la phase aqueuse sous agitation et est chauffé à une température comprise entre 55-60°C.

5. Procédé de préparation d'une crème selon la revendication 1, **caractérisé en ce que** la phase aqueuse est émulsionnée avec la phase huileuse sous agitation à une température entre 55-60°C.

6. Procédé de préparation d'une crème selon la revendication 1, **caractérisé en ce que** du sulfate de magnésium, de la glycérine et un conservateur sont de plus mélangés dans la phase aqueuse.

7. Procédé de préparation d'une crème selon la revendication précédente, **caractérisé en ce que** le sulfate de magnésium est entre 1-2 %, la glycérine est entre 1-4 % et le conservateur est entre 0,1-2 %, tous les pourcentages étant présentés en masse par rapport à la masse totale de la base de crème.

8. Procédé de préparation d'une crème selon la revendication 1, **caractérisé en ce que** du mica partiellement déstratifié est ajouté à l'émulsion de la phase aqueuse avec la phase huileuse.

9. Procédé de préparation d'une crème selon la revendication précédente, **caractérisé en ce que** du mica partiellement déstratifié est entre 1-8 % en masse en relation par rapport à la masse totale de la base de crème.

10. Procédé de préparation d'une crème selon la revendication 1, **caractérisé en ce que** l'huile végétale ajoutée dans la phase huileuse est l'huile de macadamia.

11. Procédé de préparation d'une crème selon l'une quelconque des revendications précédentes, comprenant
- le dépôt de l'émulsion de la phase aqueuse avec la phase huileuse et après cela l'addition sous agitation d'un ingrédient cosmétique actif dans une seconde phase aqueuse pour former une émulsion triple eau-huile-eau.
